# EUROPEAN PATENT SPECIFICATION

(11) **EP 4 431 122 B1**
(45) Date of publication and mention of the grant of the patent: **11.03.2026**
(21) Application number: 22891897.5
(22) Date of filing: 04.11.2022
(51) Int. Cl.: A61L 24/02, A61L 24/00, A61L 24/08, A61L 24/10, A61K 49/04, A61K 45/00, A61P 35/00

(54) **ACTIVE METAL MICROSPHERES AND COMPOSITE EMBOLIC AGENT BASED ON SAME**
AKTIVE METALLMIKROSPHÄREN UND DARAUF BASIERENDES ZUSAMMENGESETZTES EMBOLIEMITTEL
MICROSPHÈRES DE MÉTAL ACTIF ET AGENT EMBOLIQUE COMPOSITE À BASE DE CELLES-CI

(30) Priority: 12.11.2021 CN 202111342067; 13.04.2022 CN 202210384584; 30.04.2022 CN 202210469169
(43) Date of publication of application: 18.09.2024
(60) Divisional application: 26155276.4
(73) Proprietor: Chongqing Baimaitengshi Pharmaceutical Technology Co., Ltd, Chongqing 400000 (CN)
(72) Inventor: LIU, Zhuang, Suzhou, Jiangsu 215000 (CN); CHENG, Liang, Suzhou, Jiangsu 215000 (CN); GONG, Fei, Suzhou, Jiangsu 215000 (CN); YANG, Nailin, Suzhou, Jiangsu 215000 (CN)
(74) Representative: Sun, Yiming
(86) International application number: PCT/CN2022/129772
(87) International publication number: WO 2023/083102

(56) References cited:
- EP-A1- 2 365 009
- WO-A1-2016/154592
- CN-A- 1 380 106
- CN-A- 1 381 280
- CN-A- 103 536 970
- CN-A- 109 498 593
- CN-A- 110 694 100
- CN-A- 111 514 368
- CN-A- 112 245 384
- CN-B- 103 550 834
- KR-A- 20140 031 462
- KR-A- 20190 090 469
- US-A1- 2006 127 443
- US-A1- 2012 277 517
- US-E- R E47 121

## Description

### Field of the Disclosure

The present disclosure belongs to the technical field of medicines, and is in particular directed to a lipiodol-based composite embolic agent of active metal microspheres or nano-hydrides, a preparation method therefor, and applications thereof.

The present disclosure relates to active metal microspheres, and a composite embolic agent based on the active metal microspheres or nano-hydrides.

### Background technology

Cancer causes millions of deaths every year across the world, and is one of the most dangerous diseases that the mankind faces today. In particular, among the numerous cancers, liver cancer is often hardly healed by traditional anti-tumor therapies including surgical resection, organ transplantation, radiotherapy, chemotherapy or the like, due to its characteristics such as insidious onset, high degree of malignancy, strong invasiveness, rapid growth, high recurrence rate, and high mortality. In recent years, tumor interventional therapies with excellent therapeutic effect and minimal side effects have attracted widespread attention. As one of the most effective methods for targeted drug delivery, transcatheter arterial embolization (TAE) is to efficiently deliver vascular embolization materials and chemotherapeutic drugs to a tumor proximal artery by means of super-selective arterial infusion. It is considered as standard treatment for advanced solid tumors. However, due to the high heterogeneity of the liver cancer and associated blood vessels, the therapeutic response of hypovascular liver tumor tissues is hindered. Moreover, after TAE and/or the therapy combining TAE with drugs (transcatheter arterial chemoembolization, TACE), a large number of hypoxic tolerant liver cancer cells and tumor-derived stem cells (CSLCs) are accumulated at liver cancer lesions, leading to a failure in TAE. In addition, arterial embolization can cause hypoxia, weak acidity, and immunosuppressive microenvironment that promote the rapid proliferation, metastasis, and drug resistance of liver cancer cells, making it impossible to completely eradicate the liver cancer. Therefore, TAE/TACE often has problems such as incomplete tumor necrosis, recurrence/metastasis, and low patient survival rate, which greatly limit its wide application.

In recent years, immunotherapy, a therapy for activating a patient's immune system to fight against cancer, has shown great promise in the field of tumor treatment. During the process of immunotherapy, cytotoxic T lymphocytes (CTLs) are crucial for attacking tumor cells. However, the complex tumor microenvironment (TME), including abnormal blood vessels, acidic pH, hypoxia, and highlevels of reactive oxygen species (ROS), greatly limit the activity and killing ability of CTLs in solid tumors. In addition, the above physiological characteristics of TME facilitates the infiltration of various immunosuppressive cells such as myeloid-derived suppressor cells (MDSCs), M2 tumor-associated macrophages (TAMs), and regulatory T cells (T_{reg}), thereby forming an immunosuppressive microenvironment. Immunosuppressive TME is one of the major obstacles that seriously hinders the tumor immunotherapy. Therefore, it is considered as an attractive strategy to improve the efficacy of various cancer therapies, in particular the tumor immunotherapy, by modulating TME through a variety of pathways.

Hydrogen (H₂), as an endogenous gas, has a significant physiological/pathological regulatory function. Over the past ten years, hydrogen has been demonstrated to have significant anti-inflammatory and anti-tumor effects due to its high biodiffusion capacity. However, due to the low solubility of hydrogen, the current mainstream means for hydrogen delivery includes oral administration of hydrogen-rich water, injection of hydrogen-rich salt, and hydrogen inhalation, which can hardly meet the dosage requirements for effective treatment. Therefore, there is a need for the development of a novel hydrogen carrier and its delivery strategy. Reaction between active metal (for example, potassium, calcium, sodium, magnesium, aluminum, zinc, gallium, iron, manganese, and tin) microspheres or metal hydride materials (for example, calcium hydride, magnesium hydride, lithium hydride, sodium hydride, potassium hydride, strontium hydride, and cerium hydride) and water can efficiently release hydrogen, and produce hydroxide to neutralize the acidic tumor microenvironment and activate the immune system, thereby achieving microenvironmental regulation and synergistic hydrogen-immune treatment. However, these active metals or metal hydride materials show highly active chemical properties and poor stability in an aqueous system, and explosion even occurs once some active metals or metal hydrides directly contact with water. Therefore, they are generally considered difficult for direct use in tumor treatment.

CN 109 498 593 describes microparticles comprising a core of a magnesium ball which is coated with mesoporous silica. The particles are capable of sustainably and slowly releasing hydrogen in a biological medium, thereby protecting cells. By controlling the thickness of the coating layer, the release rate of the hydrogen molecules can be controlled.

### SUMMARY OF THE DISCLOSURE

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

In order to solve the above technical problems, the present disclosure provides a composite embolic agent, a preparation method therefor, and applications thereof. The composite embolic agent provided by the present disclosure is delivered to the liver cancer lesions by interventional operation to allow deposition of lipiodol on liver tumor tissues to induce embolization; in addition, active metal microspheres or hydrides release hydrogen in situ to induce hydrogen therapy for the release of hydroxyl ions to regulate immune microenvironment, thereby amplifying the embolization effect.

The target preparation involved in the present disclosure comprises magnesium microspheres or nano-calcium hydrides which are dispersed into the clinical lipiodol embolic agent to obtain a composite preparation. The said preparation has good chemical stability and moderate reaction speed even in mixture with an aqueous solution, such that hydrogen can be continuously produced in a mild way. According to the present disclosure, when used in an interventional tumor embolization procedure, the obtained lipiodol-based composite embolic agent of active metal microspheres or nano-hydrides can synchronously and continuously produce hydrogen in the tumor to implement hydrogen treatment, and further neutralizes an acidic tumor environment to improve the tumor microenvironment, thereby achieving a significantly improved efficacy compared with single lipiodol perfusion.

The first object of the present invention is to provide a lipiodol-based composite embolic agent of active metal microspheres or of nano-hydrides, comprising the active metal microspheres or nano-hydride materials and lipiodol as a dispersion protectant, wherein the active metal microspheres or the nano-hydride materials account for a mass percentage of 0.1-10% in the composite embolic agent. The lipiodol is a medical embolic agent, in which the lipiodol has the effects of dispersion and protection, and can protect the active metal and hydride to delay their reaction rate with water, allowing slow release of hydrogen and hydroxide to achieve the effect of long-term treatment.

The active metal microspheres have a size of 0.1-20 microns, and the active metal is one or more of magnesium, aluminum, zinc, gallium, manganese and tin.

The nano-hydride materials have a particle size of 5-200 nanometers, and the nano-hydride is one or more of calcium hydride, magnesium hydride, lithium hydride, sodium hydride, potassium hydride, strontium hydride and cerium hydride.

In an embodiment of the present disclosure, the 5-200 nanometers are equivalent to 0.005-0.2 microns.

In case of an overlarge particle size, the microspheres may not be well dispersed in the dispersant, and thus cannot be successfully applied clinically.

In an embodiment of the present disclosure, the active metal microspheres are prepared by a gas atomization powdering method, in which an active metal ingot is heated and molten, and then atomized and cooled to obtain the active metal microspheres.

In an embodiment of the present disclosure, the nano-hydride materials are prepared by a liquid-phase exfoliation method, in which a metal hydride is ultrasonically processed for 0.2 h to 4 h for dispersion into an exfoliation solvent which is then centrifuged to obtain the nano-hydride material.

In an embodiment of the present disclosure, the ultrasonic power is 50-100 W, and the working temperature is 10-20°C.

In an embodiment of the present disclosure, a usage ratio (M:V) (i.e., a ratio of mass (mg) to volume (mL)) of the metal hydride and the stripping solvent is 0.1:1-10:1.

In an embodiment of the present disclosure, the exfoliation solvent is one or more of N-methylpyrrolidone, dimethyl sulfoxide or polyethylene glycol 200, pyrrole, and N,N-dimethylformamide.

The second object of the present invention is to provide a method for preparing the said lipiodol-based composite embolic agent of the active metal microspheres or nano-hydrides, including the steps of: mixing and ultrasonically dispersing lipiodol and the active metal microspheres or nano-hydride materials uniformly to obtain the lipiodol-based composite embolic agent of the active metal microspheres or nano-hydrides.

The third object of the present invention the lipiodol-based composite embolic agent of the active metal microspheres or nano-hydrides as defined above for use in liver cancer embolization.

Also disclosed herein is an active metal microsphere, which has a metal activity stronger than that of hydrogen.

Further, the active metal microsphere has a particle size of 0.1-500 microns.

Further, the active metal of the active metal microsphere is one or more of magnesium, aluminum, zinc, gallium, iron, manganese, and tin.

Further, the active metal microsphere is shaped as a sphere, a mesoporous structure, or a polyhedron.

The fourth object of the present invention is to provide a composite embolic agent comprising the active metal microspheres.

The composite embolic agent further includes a dispersant.

The mass ratio of the active metal microsphere to the dispersant is 0.1:100-50:100.

Further, the dispersant is an organic phase.

Further, the dispersant is an oil phase.

Further, the dispersant is selected from one or more of lipiodol, poppyseed oil, soybean oil, and olive oil.

The fifth object of the present invention is to provide an anti-tumor agent comprising an active metal microsphere, wherein the active metal microsphere has a metal activity stronger than that of hydrogen; the active metal microsphere has a particle size of 0.1-500 microns; the active metal microsphere is prepared from an active metal, which has a metal activity greater than that of hydrogen and which is selected from at least one of magnesium, zinc, gallium, manganese, and tin.

Based on experimental validation, the inventors have found that, with the mass fraction of greater than 50% in the dispersant, the metal microsphere or the nano-hydride may not be effectively dispersed, and thus hardly be applied in practice via catheter injection.

Also disclosed herein, but not claimed, is a method for tumor treatment, in which a composite embolic agent containing metal microspheres is for use in treatment of a liver cancer, a kidney cancer, a pancreatic cancer, a lung cancer, and a pelvic malignancy.

Further disclosed herein, but not claimed, is a kit for tumor treatment, including a composite embolic agent containing metal microspheres.

Compared with the prior art, the above technical solutions provided by the present disclosure has the advantageous effects including but not limited to:
The present disclosure provides a composite embolic agent and a preparation method therefor. The said composite embolic agent is prepared by dispersing active metal microspheres or nano-hydrides into a lipiodol embolic agent at different ratios, and has good chemical stability and moderate reaction speed even in mixture with an aqueous solution, such that hydrogen can be continuously produced in a mild way, which solves the problem of uncontrollable violent reaction between the active metal microspheres or nano-hydride materials and water; In the present disclosure, the obtained lipiodol-based composite embolic agent of active metal microspheres or nano-hydrides is locally delivered to liver tumor tissues by interventional operation to allow deposition of the lipiodol on liver tumor tissues to induce embolization; in addition, the active metal microspheres or hydrides continuously produce hydrogen in the tumor to achieve the effect of hydrogen treatment, and the hydroxides released at the same time neutralize the weak acidic tumor microenvironment to improve the tumor microenvironment, thereby amplifying the embolization effect and achieving a significantly improved efficacy compared with single lipiodol perfusion;

The target preparation involved in the present disclosure comprises magnesium microspheres or nano-calcium hydrides, which are dispersed into the clinical lipiodol embolic agent to obtain a composite preparation with good chemical stability;

The method for preparing the composite embolic agent according to the present disclosure is simple and convenient for clinical use.

### BRIEF DESCRIPTION OF THE DRAWINGS

For easier and clear understanding of the content of the present disclosure, the present disclosure is further described in detail below according to the specific embodiments of the present disclosure in combination with the accompanying drawings, in which
FIG. 1 shows microphotographs of obtained metal magnesium microspheres according to Embodiment 1 of the present disclosure.
FIG. 2 shows a photo for evaluating hydrogen release of the obtained metal magnesium microspheres in different buffers according to Embodiment 1 of the present disclosure.
FIG. 3 shows a transmission electron microscope (TEM) image of obtained nano-calcium hydride according to Embodiment 2 of the present disclosure.
FIG. 4 shows an X-ray diffraction (XRD) graph of the obtained nano-calcium hydride according to Embodiment 2 of the present disclosure.
FIG. 5 shows an optical photo of an obtained metal magnesium microsphere-lipiodol composite embolic agent according to Embodiment 1 of the present disclosure.
FIG. 6 shows an optical photo of an obtained nano-calcium hydride-lipiodol composite embolic agent according to Embodiment 2 of the present disclosure.
FIG. 7 shows the evaluation of hydrogen release of the obtained calcium hydride-lipiodol composite embolic agent according to Embodiment 2 of the present disclosure.
FIG. 8 shows a schematic diagram of an interventional operation process for rabbit liver cancer according to Application Example 1 of the present disclosure.
FIG. 9 shows the DSA imaging of liver cancer lesions in the rabbit interventional operation process according to Application Example 1 of the present disclosure.
FIG. 10 shows the CT imaging of liver cancer lesions before and after the embolization operation in rabbits using the obtained magnesium microsphere-lipiodol composite embolic agent according to Embodiment 1 of the present disclosure.
FIG. 11 shows the CT imaging of liver cancer lesions before and after the embolization operation in rabbits using the obtained nano-calcium hydride-lipiodol composite embolic agent according to Embodiment 2 of the present disclosure.
FIG. 12 shows the ¹⁸F-FDG PET imaging of the liver cancer lesions before and after the embolization operation in rabbits using the obtained nano-calcium hydride-lipiodol composite embolic agent according to Embodiment 2 of the present disclosure.
FIG. 13 shows the strength of ¹⁸F-FDG at the liver cancer lesions before and after the embolization operation in rabbits using the obtained nano-calcium hydride-lipiodol composite embolic agent according to Embodiment 2 of the present disclosure.
FIG. 14 shows the immunohistochemical analysis of the ex-vivo liver cancer lesions seven days after the embolization operation in rabbits using the obtained nano-calcium hydride-lipiodol composite embolic agent according to Embodiment 2 of the present disclosure.
FIG. 15 shows the apoptosis levels of the ex-vivo liver cancer lesions seven days after the embolization operation in rabbits using the obtained nano-calcium hydride-lipiodol composite embolic agent according to Embodiment 2 of the present disclosure.
FIG. 16 shows the proliferative activity of the ex-vivo liver cancer lesions seven days after the embolization operation in rabbits using the obtained nano-calcium hydride-lipiodol composite embolic agent according to Embodiment 2 of the present disclosure.
FIG. 17 shows a graph of particle size distribution of a magnesium-platinum metal composite structure according to Embodiment 3 of the present disclosure.
FIG. 18 shows a statistical diagram of hydrogen yields detected according to Embodiment 4 of the present disclosure, from the magnesium microspheres and the Mg@Pt composite structure obtained in Embodiment 3 in 96% ethanol solution.
FIG. 19 shows a mouse tumor growth curve obtained according to Embodiment 6 of the present disclosure, during mouse tumor treatment using the Mg@Pt metal composite structure-lipiodol composite embolic agent prepared in Embodiment 3.
FIG. 20 shows electron scanning microscope images of the magnesium microspheres and Mg@Pt composite structure as raw materials according to Embodiment 7 of the present disclosure.
FIG. 21 shows an XRD powder diffraction pattern of the magnesium-platinum metal composite structure prepared according to Embodiment 8 of the present disclosure.
FIG. 22 shows a curve graph of the total hydrogen yield detected according to Embodiment 9 of the present disclosure, from the Mg@Pt metal composite structure prepared in Embodiment 8 in a phosphate buffer.
FIG. 23 shows a statistical graph of the particle size distribution of large-diameter magnesium microspheres according to Embodiment 15 of the present disclosure.
FIG. 24 shows a curve graph of the total hydrogen yield detected by gas chromatograph according to Embodiment 16 of the present disclosure, from the magnesium microspheres in Embodiment 15 in buffers under different pH conditions.
FIG. 25 shows a graph of particle size distribution of magnesium microspheres according to Embodiment 18 of the present disclosure.
FIG. 26 shows a curve graph of the content of hydrogen produced over time by the magnesium microspheres in buffers with different pH values, detected according to Embodiment 18 of the present disclosure.
FIG. 27 shows images of rabbit liver tissues after the establishment of a rabbit liver cancer model and after the use of a composite embolic agent in the rabbit liver cancer model according to Embodiment 19, with the left image taken after the establishment of the rabbit liver cancer model in Embodiment 19, and the right image taken after the use of the composite embolic agent in the rabbit liver cancer model, which indicates the vascular embolization.
FIG. 28 shows an ultrasound image of lesions before and after delivery of the magnesium microspheres according to Embodiment 19 of the present disclosure.
FIG. 29 shows the CT image data at different times before and after treatment according to Embodiment 19.1 and Comparative Example 19.2 of the present disclosure.
FIG. 30 shows an X-ray diffraction pattern of the manganese-platinum metal composite structure of the present disclosure.
FIG. 31 shows a statistical graph of the content of hydrogen produced by the metal manganese and metal composite structure in phosphate buffer at different time points according to the present disclosure.
FIG. 32 shows tumor growth curves of mice in different groups according to Embodiment 22 of the present disclosure.
FIG. 33 shows a statistical graph of residual magnesium at the mouse tumor site at different times after intratumoral injection of magnesium microsphere-lipiodol composite embolic agent according to Embodiment 23 of the present disclosure.
FIG. 34 shows a tendency chart of pH changes over time in mouse tumors according to Embodiment 24 of the present disclosure.
FIG. 35 shows a statistical graph of the contents of hydrogen produced by the magnesium-platinum metal composite structures at different ratios in a solution within 24 hours according to Embodiment 25 of the present disclosure; and
FIG. 36 shows a statistical graph of the content of hydrogen produced by the manganese-platinum metal composite structure at different ratios in a solution within 24 hours according to Embodiment 26 of the present disclosure.

### DETAILED DESCRIPTION OF THE PREFERRED

### EMBODIMENTS

The present disclosure will be further described below in combination with the accompanying drawings and specific embodiments, such that those skilled in the art can better understand and implement the present disclosure, but the embodiments provided are not intended to limit the present disclosure.

For more detailed description of the above technical solutions of the present disclosure, specific embodiments are listed below to demonstrate the technical effect. It should be emphasized that these embodiments are for the purpose of illustrating the present disclosure instead of limiting the scope of the present disclosure.

As indicated in the specification and claims, unless otherwise explicitly indicating exceptions in the context, the terms "a", "an", "the" and/or "said" do not refer in particular to a singular form, and may include a plural form. The terms "comprise" and "include" only imply the inclusion of explicitly identified elements, which do not constitute an exclusive list, and a device may also include other elements. The term "an embodiment" refers to "at least one embodiment"; and the term "another embodiment" refers to "at least one additional embodiment". Relevant definitions of other terms will be given in the description below.

The detection apparatuses and methods used in the embodiments in the specification are as follows:
Morphological characterization: transmission electron microscope (FEI Tecnai F20 TEM), scanning electron microscope (ZEISS Sigma);
Elemental analysis: X-ray powder diffractometer (PANalytical X-ray diffractometer);
Determination of hydrogen production efficiency: gas chromatograph (SIEMENS MAXUM II);
In vivo angiography to detect embolization: DAS digital subtraction angiograph (PHILIPS FD20);
In vivo angiography to detect tumor size:
   (1) CT imager (PHILIPS Access CT), using CT imaging enhanced with ioversol contrast agent to evaluate the efficacy;
   (2) PET-CT imager (GEMINI TF PET/CT), using ¹⁸F-FDG as a nuclide probe to evaluate the effect of interventional embolization by means of positron emission tomography (PET)/CT imaging;
Method for evaluating tumor apoptosis and proliferation: staining tumor slices using fluorescently labeled TUNEL or Ki67 proteins, and observing and counting the proportion of TUNEL- or Ki67-positive cells in the tumor cells in the field of view, the proportion being the apoptosis or proliferation rate of tumor cells.

The tumor cell line in the embodiment is the H22 tumor cell line.

The present disclosure provides a composite embolic agent, comprising: active metal microspheres or nano-hydride materials and lipiodol as a dispersion protectant.

The active metal microspheres are one or more of magnesium, aluminum, zinc, gallium, manganese, and tin. The above-mentioned active metal microspheres are in the order of microns, with the particle size of 0.1-20 microns. The active metal microspheres are prepared from an active metal ingot by a gas atomization powdering method. The hydrides are one or more of cationic metal hydrides selected from calcium hydride, magnesium hydride, lithium hydride, sodium hydride, potassium hydride, strontium hydride, and cerium hydride.

The above-mentioned hydride materials are all nanoscale, with the particle size of 5-200 nanometers. The nano-hydride materials are obtained by exfoliating the raw material hydrides by a liquid phase exfoliation method, with the specific reaction conditions as follows: dispersing a metal hydride raw material into N-methylpyrrolidone as an exfoliation solvent, performing ultrasonic exfoliation for 20 min, and performing ultracentrifugation to obtain a nano-hydride product.

Subsequently, the active metal microspheres or nano-hydride particles are mixed with lipiodol and ultrasonically dispersed uniformly to obtain the composite embolic agent. The mass percentage of the active metal microspheres or nano-hydride in the composite embolic agent is 0.1% to 10%. The prepared composite embolic agent is locally delivered to liver tumor tissues by interventional operation to allow deposition of lipiodol on the liver tumor tissue to induce embolization; in addition, active metal microspheres or hydrides release hydrogen in situ to induce hydrogen therapy and release hydroxide to regulate tumor microenvironment, thereby amplifying the embolization therapy effect. The composite embolic agent as defined in the present disclosure regulates tumor microenvironment by in situ release of hydrogen, hydroxide, or the like from the active metal microspheres or metal hydrides, showing a better combined embolization therapy effect than simple lipiodol embolization.

In the various embodiments described herein below, embodiments 3-14, 20-22 and 25-27 are to be considered as reference-embodiments, not falling within the scope of the claimed invention. Also the comparative examples are reference-embodiments, not falling within the scope of the claims.

### Embodiment 1: Preparation of Magnesium Microsphere-Lipiodol Composite Embolic Agent

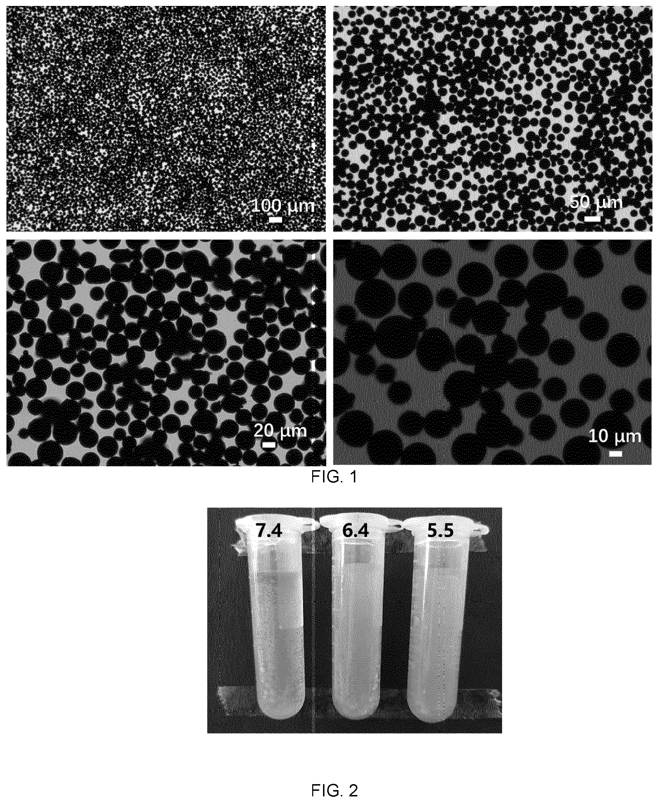

A metal magnesium ingot was put into a gas atomization powdering apparatus, and then heated to 700°C to form a stable flowing liquid, and then the liquid magnesium was cooled with a high-speed inert argon gas at a flow of Mach 2-2.5 to obtain magnesium powders, which was let pas through a 1000-mesh sieve to obtain magnesium microspheres of 10 microns. The structure of the obtained magnesium microspheres was characterized, with the results shown in FIG. 1-FIG. 2.

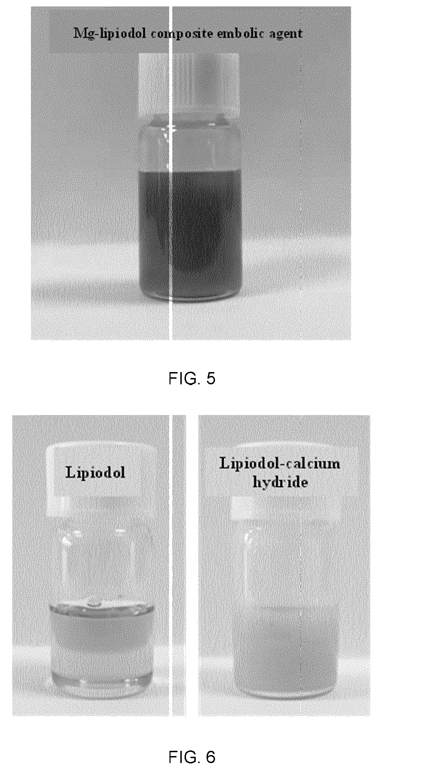

The magnesium microspheres prepared above were dispersed into a commercial lipiodol embolic agent at a mass fraction of 1%, by sonication or by shaking to obtain a magnesium microsphere-lipiodol composite embolic agent. The composite embolic agent can be customized as required, with the mass ratio of the magnesium microspheres being 1%. The commercial lipiodol used as a dispersant is a light yellow clear transparent liquid, and the prepared magnesium microsphere-lipiodol composite embolic agent is a black-yellow liquid, as shown in the optical photo in FIG. 5.

FIG. 1 shows a microphotograph of the prepared magnesium microspheres, from which the magnesium microspheres are found to have uniform morphology, with the particle size of 10 microns.

FIG. 2 shows the hydrogen release rate of the magnesium microspheres in buffers with different pH value (7.4, 6.4, 5.5). It can be found that the higher the acidity of the solution, the greater the amount of bubbles generated, indicating higher hydrogen release rate.

### Embodiment 2: Preparation of Calcium Hydride-Lipiodol Composite Embolic Agent

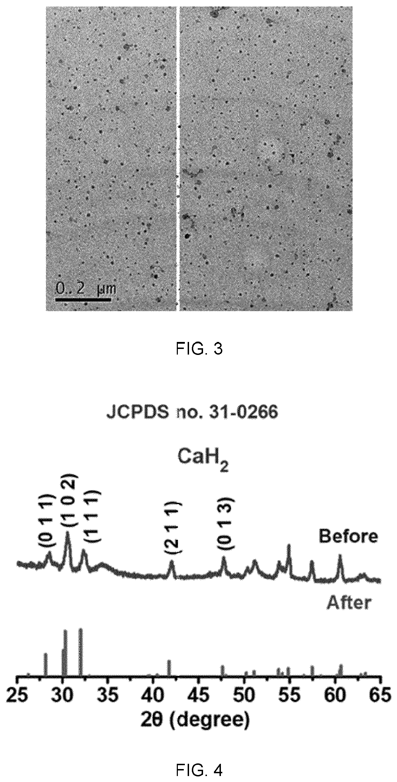

Calcium hydride powder and N-methylpyrrolidone as an organic solvent were added into a reactor in a ratio (M:V, i.e., mass (mg):volume (mL)) of 10:1. The resulting mixture was shaken vigorously to uniformly disperse the calcium hydride powder into the N-methylpyrrolidone. The mixed dispersion system was placed in an ultrasonic cleaner, and was sonicated for 0.3 h. The working power was 100 W and the working temperature was 15°C in the ultrasonic cleaner. The treated reaction system was ultracentrifuged to obtain precipitates, which were washed with ethanol several times to obtain calcium hydride nanoparticles. The structure of the obtained calcium hydride nanoparticles was characterized, with the results shown in FIG. 3-FIG. 4.

FIG. 3 shows a transmission electron microscope image of the prepared calcium hydride nanoparticles, from which the prepared product is found to have uniform morphology, with the particle size of 7.9 nanometers.

FIG. 4 shows the X-ray diffraction pattern of the synthesized nano-calcium hydride, with the abscissa indicating the degree of 2θ angle, and the ordinate indicating the intensity. The 2θ angles of the characteristic diffraction peaks of the calcium hydride nanoparticles are 28.18, 30.38, 32.04, 41.72, and 47.56, which correspond to diffraction crystal planes (011), (102), (111), (211), and (013), respectively. The above diffraction peak data are consistent with the standard X-ray diffraction data of calcium hydride (JCPDS no. 31-0266), which can demonstrate that the synthesis method described in the present disclosure can successfully prepare the calcium hydride nanoparticles.

The nano-calcium hydride powder prepared above was dispersed into a commercial lipiodol embolic agent at the mass percentage of 0.4%, and then dispersed by sonication or by shaking to obtain the calcium hydride-lipiodol composite embolic agent. The composite embolic agent can be customized as required, with the mass ratio of the calcium hydride being 0.4%. The commercial lipiodol used as a dispersant is a light yellow clear transparent liquid, as shown in the left optical photo in FIG. 6, and the prepared nano-calcium hydride-lipiodol composite embolic agent is a milky white liquid, as shown in the right optical photo in FIG. 6.

### Test Examples: Testing of Hydrogen Release Performance of Nano-Calcium Hydride-Lipiodol Composite Embolic Agent Obtained in Embodiment 2.

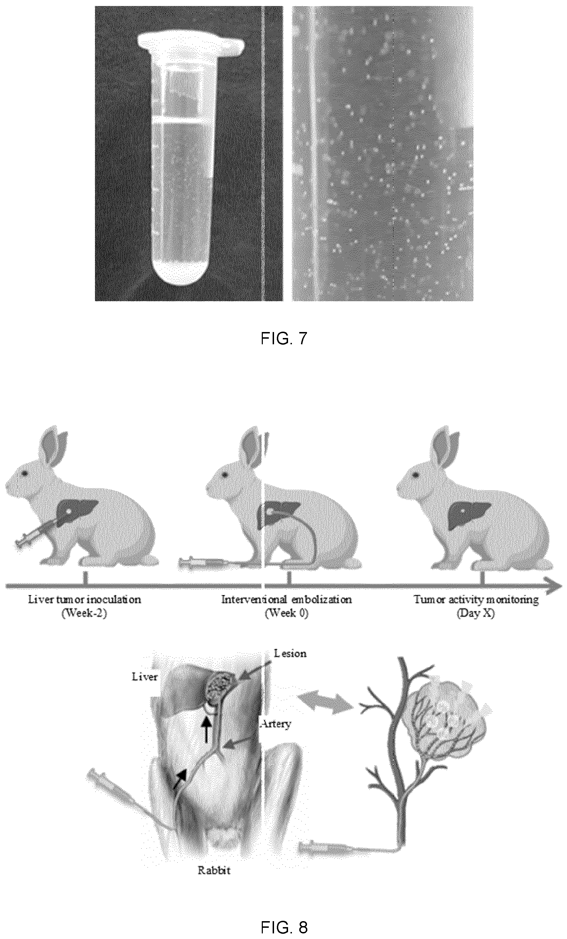

50 mL of composite embolic agent was dropwise added to an EP tube containing 3 mL of water, which was monitored in real time for hydrogen production. As shown in the optical photos in FIG. 7, a large number of bubbles were produced in the EP tube, which indicates good in situ hydrogen release performance of the nano-calcium hydride-lipiodol composite embolic agent, and demonstrates the stability and practicability of the composite embolic agent.

### Application Example 1: Application of Magnesium Microsphere-Lipiodol Composite Embolic Agent Obtained in Embodiment 1 to Interventional Embolization for Rabbit Liver Cancer

With the guidance of CT imaging, the VX2 liver tumor tissue was directly embedded in the left lobe of the liver, and the tumor could be formed after two weeks to establish a rabbit liver cancer in situ model. The tumor-bearing rabbits were randomly divided into three groups: (1) a control group; (2) a lipiodol group; (3) a magnesium microsphere-lipiodol composite embolic agent group. As shown in the detailed therapeutic regimen in FIG. 8, a 2.0-F microcatheter and a coaxial guide wire cannula were inserted into the left hepatic artery containing tumor lesions via the femoral artery under the guidance of DSA. Then, 0.3 mL of the lipiodol or magnesium microspheres-lipiodol composite embolic agent was slowly injected into the left hepatic artery via a microcatheter while avoiding reflux. After operation, the efficacy was monitored by CT imaging and enhanced CT. With the deposition of lipiodol on the liver cancer lesions, the magnesium microspheres were locally delivered to the lesions and slowly reacted with water to release hydrogen and magnesium hydroxide in the liver tumor. The magnesium hydroxide could neutralize the weakly acidic tumor microenvironment, the hydrogen could trigger hydrogen therapy, and the combination of the two could enhance the therapeutic effect of lipiodol embolization.

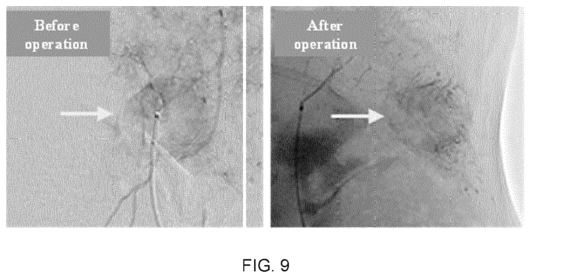

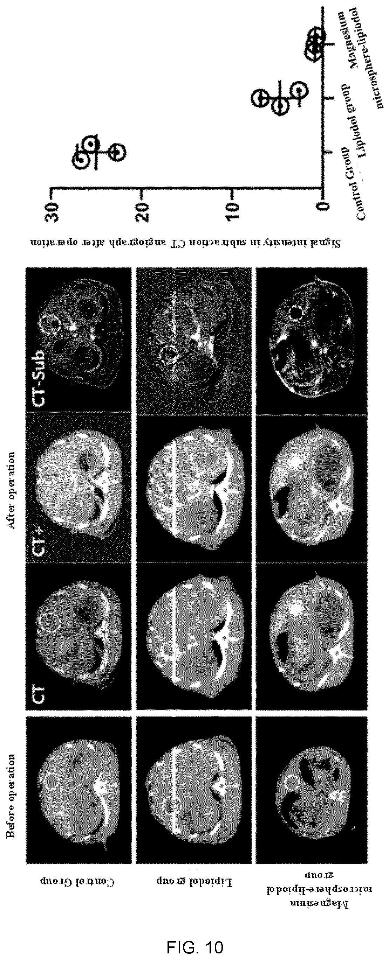

The specific experimental results are shown in FIG. 9 and FIG. 10. Firstly, as can be seen from FIG. 9, the liver cancer lesions on the left liver lobe of the tumor-bearing rabbit had the size of 1.0-1.5 cm, indicating successful establishment of the liver cancer model. After the femoral artery interventional operation, a large amount of lipiodol was found deposited in the rabbit liver tumors in the lipiodol group and the magnesium microsphere-lipiodol composite embolic agent group, indicating successful interventional embolization. One week after the interventional operation, the efficacy was evaluated using CT imaging enhanced with ioversol contrast agent according to the present disclosure. The results are shown in FIG. 10. In the control group, the tumor growth was fast, with the size increased by 3.3 times, and the subtraction CT angiography showed that the CT signal intensity at the lesion region was 26.68, which was evaluated as a progressive disease according to the modified Response Evaluation Criteria in Solid Tumor (mRECIST). In the simple lipiodol embolization group, the tumor growth was inhibited, without any increase in volume; but the enhanced CT imaging showed that some tissues of the lesion were still enhanced (the subtraction CT angiography showed that the lesion was still partially enhanced in white, and the CT signal intensity was 3.63), indicating that the simple lipiodol embolization at the current dose could not achieve complete tumor killing; and it was evaluated as a partial response according to mRECIST. However, the present disclosure did not find any enhancement at the tumor site in the magnesium microsphere-lipiodol composite embolic agent group (the subtraction CT angiography showed that the lesion was enhanced in white, and the CT signal intensity was 0.86), indicating an excellent efficacy of this composite embolic agent, and it was evaluated as a complete response according to mRECIST.

### Application Example 2: Application of Calcium Hydride-Lipiodol Composite Embolic Agent Obtained in Embodiment 2 to Interventional Embolization for Rabbit Liver Cancer

Like the modeling scheme described above, with the guidance of CT imaging, the VX2 liver tumor tissue was directly embedded in the left lobe of the liver, and the tumor was formed after two weeks to obtain a rabbit liver cancer in situ model. The tumor-bearing rabbits were randomly divided into three groups: (1) a control group; (2) a lipiodol group; (3) a nano-calcium hydride-lipiodol composite embolic agent group. Afterwards, a 2.0-F microcatheter and a coaxial guide wire cannula were inserted into the left hepatic artery containing tumor lesions via the femoral artery under the guidance of DSA. Then, 0.3 mL of the lipiodol or nano-calcium hydride-lipiodol composite embolic agent was slowly injected into the left hepatic artery via a microcatheter while avoiding reflux. After operation, the efficacy was monitored by CT imaging.

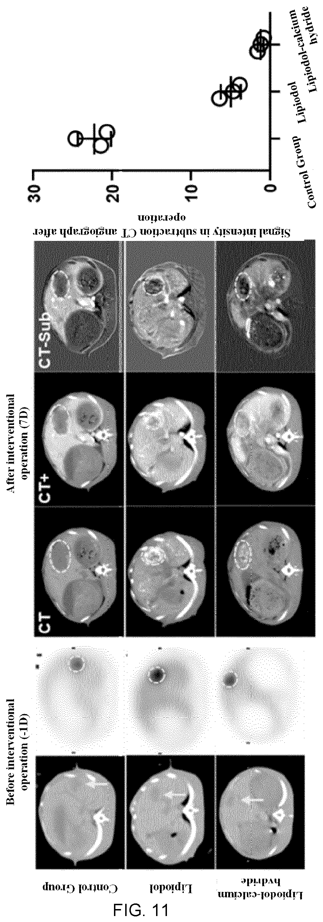

The liver cancer lesions on the left liver lobe of the tumor-bearing rabbit had the size of 1.0-1.5 cm. After the femoral artery interventional operation, a large amount of lipiodol was found deposited in the rabbit liver tumors in the lipiodol group and the nano-calcium hydride-lipiodol composite embolic agent group, indicating successful interventional embolization. One week after the interventional operation, the efficacy was evaluated using CT imaging enhanced with ioversol contrast agent according to the present disclosure. The results were shown in FIG. 11. In the control group, the tumor growth was fast, with the size increased by 10 times, and the subtraction CT angiography showed that the CT signal intensity at the lesion region was 21.46, which was evaluated as a progressive disease according to mRECIST. In the simple lipiodol embolization group, the tumor growth was inhibited, without any increase in volume; but the enhanced CT imaging showed that some tissues of the lesion were still enhanced (the subtraction CT angiography showed that the lesion was still partially enhanced in white, and the CT signal intensity was 3.86), indicating that the simple lipiodol embolization at the current dose could not achieve complete tumor killing; and it was evaluated as a partial response according to mRECIST. However, the present disclosure did not find any enhancement at the tumor site in the nano-calcium hydride-lipiodol composite embolic agent group (the subtraction CT angiography showed that the lesion was enhanced in white, and the CT signal intensity was 1.35), indicating an excellent efficacy of this composite embolic agent, and it was evaluated as a complete response according to mRECIST.

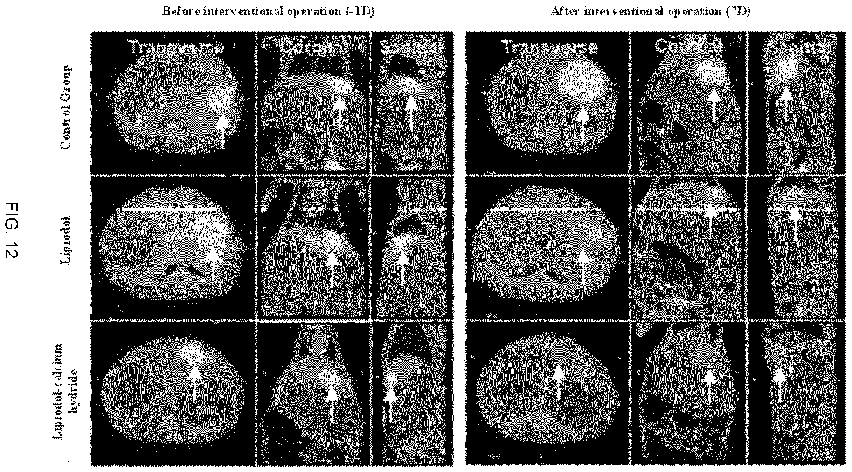

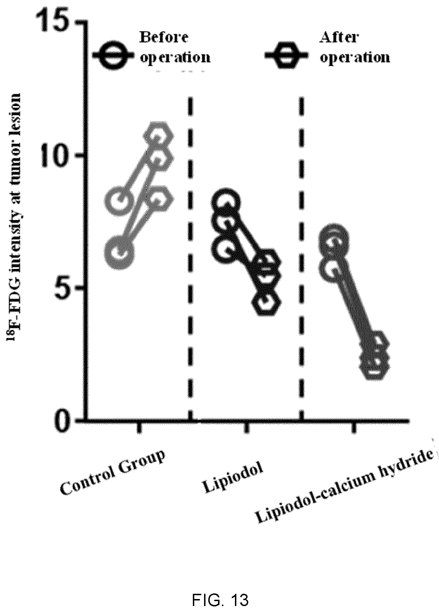

Due to the overexpression of glucose transporter-3 on the surface of tumor cells, fluorinated glucose was accumulated significantly on the tumor tissues, providing a strong guarantee for the clinical diagnosis of tumors. Therefore, the present disclosure evaluated the effect of interventional embolization by means of positron emission tomography (PET)/CT imaging using ¹⁸F-FDG as a nuclide probe. As shown in FIG. 12 and FIG. 13, PET/CT imaging showed strong ¹⁸F-FDG signals at the liver cancer lesions before the operation, with an average ¹⁸F intensity of 6.95, indicating very high biological activity. Seven days after operation, in the untreated control group, the tumor volume increased, the high metabolism region increased, and the average ¹⁸F intensity increased to 9.64; while in the simple lipiodol group, the ¹⁸F-FDG signal decreased significantly, and the average ¹⁸F intensity decreased to 5.26, which was still at a moderate level. It was worth noting that in the nano-calcium hydride-lipiodol composite embolic agent group, the ¹⁸F-FDG signal at the rabbit liver cancer sites completely disappeared, and the average ¹⁸F intensity was 2.40, which was no different from that of normal tissues (the average ¹⁸F intensity of which was 2.20), demonstrating the nano-calcium hydride-lipiodol composite embolic agent had the best combined efficacy. With the deposition of lipiodol, the nano-calcium hydrides were delivered in situ to the tumor sites and slowly releases hydrogen and calcium hydroxide in situ to enhance the effect of interventional embolization, indicating the conversion potential of the nano-calcium hydride-lipiodol composite embolic agent.

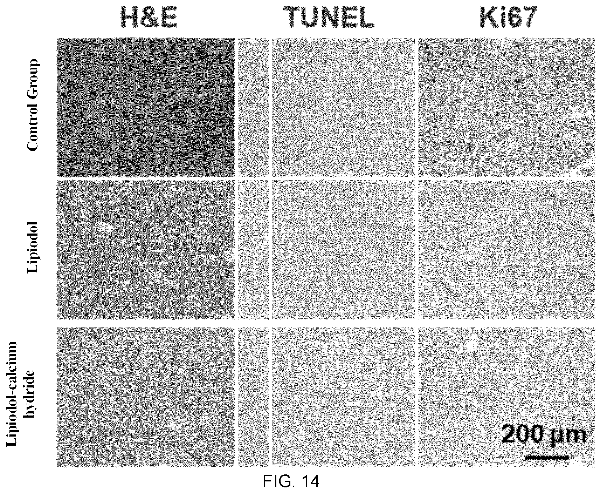

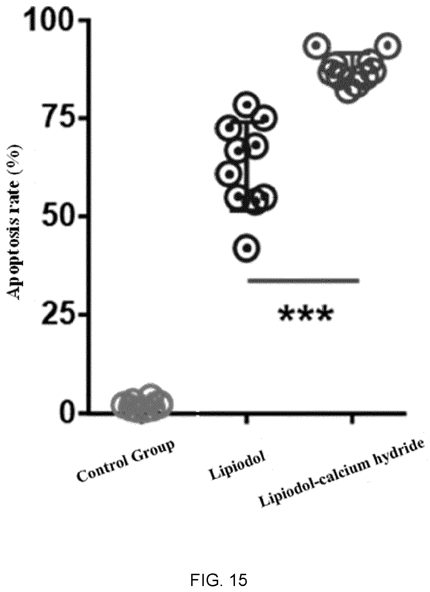

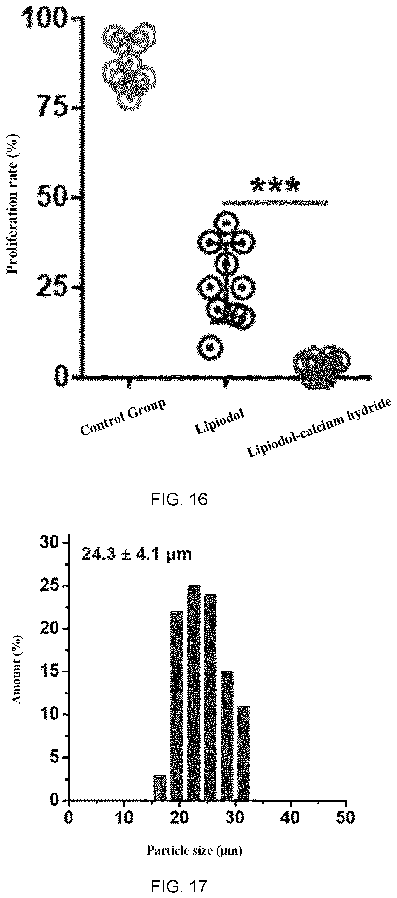

To further demonstrate the effect of embolization, according to the present disclosure, liver tumor tissues were isolated and sliced, stained with H&E, TUNEL and Ki67, and undergone histopathological analysis to evaluate tumor tissue damage, cell apoptosis and cell proliferation. The results were shown in FIG. 14. Compared with the simple lipiodol and the control group, the nano-calcium hydride-lipiodol composite embolic agent group showed the highest tumor cell apoptosis rate (control group: 2.1%; simple lipiodol group: 62.7%; and nano-calcium hydride-lipiodol composite embolic agent group: 87.6%) and the lowest cell proliferation level (control group: 87.5%; simple lipiodol group: 26.1%; and nano-calcium hydride-lipiodol composite embolic agent group: 2.9%) (FIG. 15 and FIG. 16), demonstrating that the composite embolic agent had an excellent efficacy as a novel embolic agent, and was a medicament with great potential for clinical transformation.

### Reference-embodiment 3: Magnesium-Platinum Metal Composite Structure (Mg@Pt Composite Structure)

A magnesium-platinum metal composite structure comprising a magnesium core structure and platinum particles bound on the surface of the magnesium core structure. FIG. 17 is a diagram of particle size distribution of the magnesium-platinum metal composite structure, in which the particle size distribution of magnesium-platinum metal composite microspheres is about 25 microns. The method for preparing the magnesium-platinum metal composite structure is as follows.

0.1% chloroplatinic acid in ethanol solution was prepared; 100 mg of magnesium microspheres (with the particle size of 24 microns) were added to the above-mentioned chloroplatinic acid in ethanol solution and then sonicated, until the original off-white magnesium microspheres changed to black brown; after the ethanol volatilized completely, the magnesium-platinum metal composite structure (Mg@Pt composite structure) was obtained.

The corresponding concentration of chloroplatinic acid was determined based on the quantity, shape, and particle size of the particles with the magnesium core structure.

### Reference-embodiment 4: Testing of Hydrogen Production Rate of Magnesium-Platinum Metal Composite Structure of Reference-embodiment 3

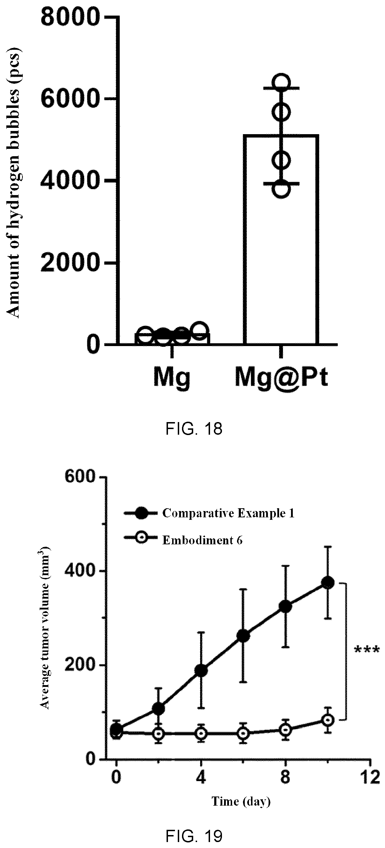

For the convenience of comparing the hydrogen production efficiency between the magnesium microspheres and the magnesium-platinum composite structure, 20 mg of magnesium microspheres and the magnesium-platinum metal composite structure obtained in Reference-embodiment 3 were placed in 96% ethanol solution separately, and the hydrogen production in the two samples was observed based on hydrogen bubbles produced after about 10 s of reaction. The results in FIG. 18 show that a large number of bubbles appeared in the ethanol solution containing the magnesium-platinum metal composite structure. The amount of bubbles produced in the same period of time was counted, and it was found that the magnesium-platinum metal composite structure had 200 times higher gas production efficiency in the ethanol solution than the simple Mg microspheres, indicating that the magnesium-platinum metal composite structure had higher hydrogen production efficiency than that of the active metal element. The reason is that, in the magnesium-platinum metal composite structure, a galvanic cells composite structure can be formed between the inert metal platinum and the active metal magnesium, which increases the hydrogen production efficiency of the active metal, magnesium.

Hydrogen can selectively clear highly cytotoxic reactive oxygen species [hydroxyl radicals (·OH) and peroxynitrite anions (ONOO⁻)], disrupt the intracellular redox equilibrium, and induce tumor cell apoptosis while retaining physiologically necessary ROS for normal cell signal transduction, thereby resulting in low side and toxic effects. Therefore, the magnesium-platinum metal composite structure is more efficient in hydrogen production, which helps to increase the hydrogen content in the tumor microenvironment, thereby enabling more effective regulation of the tumor microenvironment. The composite embolic agent prepared with the magnesium-platinum metal composite structure have better efficacy for tumor treatment.

### Reference-embodiment 5: Composite Embolic Agent

A composite embolic agent comprising the magnesium-platinum metal composite structure of Reference-embodiment 3 and lipiodol. Simple lipiodol was a light yellow clear transparent liquid in appearance, and the composite embolic agent was a milky white liquid. The composite embolic agent was prepared by the method including: dispersing the magnesium-platinum metal composite structure prepared in Reference-embodiment 3 into lipiodol (commercially available), and performing ultrasonic processing or shaking for uniform dispersion, to obtain a magnesium-platinum metal composite structure-lipiodol composite embolic agent. The mass fraction of the magnesium-platinum metal composite structure in lipiodol was 6.0%.

### Reference-embodiment 6: Composite Embolic Agent for Use in Tumor Treatment in Mice

The magnesium-platinum metal composite structure-lipiodol composite embolic agent prepared in Reference-embodiment 5 was used for tumor treatment in mice.

Firstly, H22 tumor cells were inoculated on the right backs of mice to establish mouse subcutaneous liver tumor models; after the tumors in the mice grew to about 80 mm³, the mice were randomly divided into two groups, i.e. the lipiodol embolization group and the magnesium-platinum metal composite structure-lipiodol composite embolic agent group; and the mouse tumor growth was measured and recorded to draw a curve graph of mouse tumor growth.

### Comparative Example 1 (not according to the claimed invention): Lipiodol Embolic Agent for Use in Tumor Treatment in Mice

Comparative Example 1 differed from Reference-embodiment 6 in that the mice were treated with the lipiodol embolic agent before measuring and recording mouse tumor growth. The treatment method and testing method were the same as those in Reference-embodiment 6.

The results were shown in FIG. 19. In Embodiment 6, the tumors in the mice were treated with the Mg@Pt metal composite structure-lipiodol composite embolic agent, and the average tumor volume in the mice was about 50 cubic millimeters on Day 10 after treatment; while in Comparative Example 1, the mice were treated with the lipiodol embolic agent, and the average tumor volume in the mice was close to 400 cubic millimeters on Day 10.

Therefore, compared with Comparative Example 1, the Mg@Pt metal composite structure-lipiodol composite embolic agent of Reference-embodiment 6 showed a significantly improved efficacy on tumors. *** shown in FIG. 19 refers to a p value of < 0.001 that indicates statistical significance. A smaller p value indicates more significant difference between groups. The p value calculated herein showed that there was a significant difference between Embodiment 6 and Comparative Example 1, therefore, the Mg@Pt metal composite structure-lipiodol composite embolic agent showed a significantly enhanced tumor embolization effect compared with the traditional lipiodol embolic agent.

### Reference-embodiment 7: Magnesium-Platinum Metal Composite Structure and Method for Preparing Same

A magnesium-platinum metal composite structure comprising a magnesium core structure and platinum particles bound on the surface of the magnesium core structure. The core structure is spheric, and the particle size distribution of magnesium-platinum metal composite microspheres is about 350 microns. The method for preparing the magnesium-platinum metal composite structure is as follows.

0.1% chloroplatinic acid in ethanol solution was prepared; 100 mg of magnesium microspheres (with the particle size of about 350 microns) were added to the above-mentioned chloroplatinic acid in ethanol solution and then sonicated until the original off-white magnesium microspheres changed to black brown. After the reaction, the liquid was removed, and the residual ethanol volatilized completely to obtain the magnesium-platinum metal composite structure.

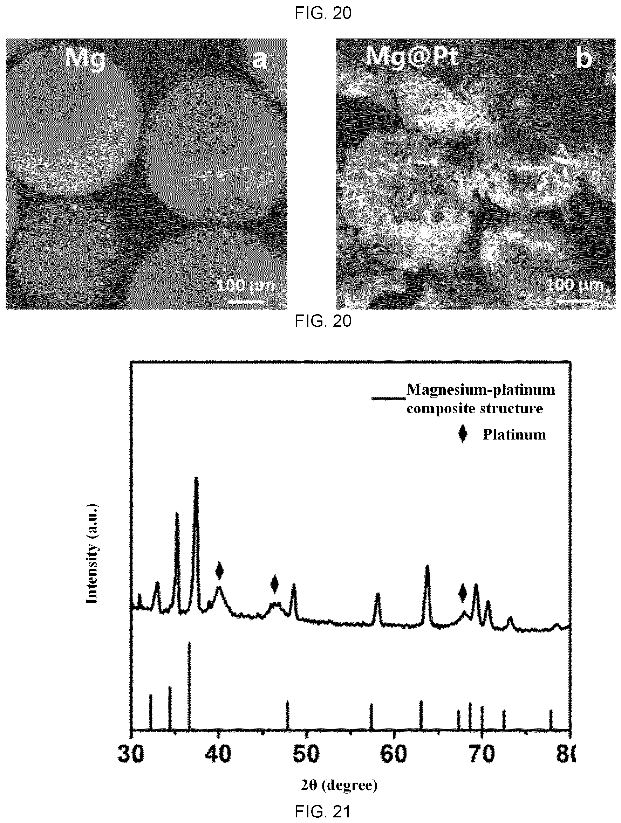

The raw material magnesium microspheres and the magnesium-platinum metal composite structure in Reference-embodiment 4 were morphologically characterized using an optical microscope, with the results shown in FIG. 20. In FIG. 20, "a" was the electron scanning microscope image of the raw material magnesium microspheres, and "b" was the electron scanning microscope image of the magnesium-platinum metal composite structure.

Comparing a and b in FIG. 20, it can be seen that the surfaces of the microspheres of the magnesium-platinum metal composite structure were rougher, which were the platinum attached to the surfaces of the magnesium microspheres. This indicates that the platinum adheres to the surfaces of the magnesium microspheres by means of a reduction reaction to form the magnesium-platinum metal composite structure.

### Reference-embodiment 8: Magnesium-Platinum Metal Composite Structure

Reference-embodiment 8 differed from Reference-embodiment 7 in that the particle size of the raw material magnesium microspheres was about 500 microns. The magnesium-platinum metal composite structure was prepared by the method of Reference-embodiment 7.

### Reference-embodiment 9: Magnesium-Platinum Metal Composite Structure

A magnesium-platinum metal composite structure comprising a magnesium core structure and platinum particles bound on the surface of the magnesium core structure. The core structure was rod-like. The method for preparing the rod-like magnesium-platinum metal composite structure is as follows.

A magnesium rod (diameter: 0.5 mm, length: 2 mm, commercially available) was placed in 2 mL of a neutral solution (sodium chloroplatinate solution) containing 0.3% chloroplatinate ions (PtCl₆²⁻) and reacted for 1 min; then a rod-like magnesium-platinum metal composite structure was taken out and washed three times with absolute ethanol to remove unreacted ions on the surface; and the prepared rod-like magnesium-platinum composite structure was sealed and preserved in absolute ethanol. According to the principle of in-situ replacement reduction, the magnesium-platinum metal composite structure was formed on the surface of magnesium, and platinum nanoparticles were reduced on the surface of the magnesium rod by PtCl₆²⁻, thereby obtaining the rod-like magnesium-platinum metal composite structure.

FIG. 21 was the XRD pattern of the rod-like magnesium-platinum metal composite structure prepared in Embodiment 9. In FIG. 21, the vertical line on the abscissa axis indicated the standard XRD signal peak position and relative intensity of magnesium elements, the curve graph showed the XRD signal pattern of the rod-like magnesium-platinum metal composite structure, and the diamond was marked as the XRD signal peak position of the platinum element. As can be seen from FIG. 21, in the XRD pattern of the rod-like magnesium-platinum metal composite structure, the signals of the elemental magnesium were consistent with the standard signal peak positions of the magnesium element. In addition to the strong magnesium signal, there were also platinum signal peaks, which further demonstrated that the platinum nanoparticles were successfully grown on the surface of the magnesium rod to obtain the rod-like magnesium-platinum metal composite structure.

### Reference-embodiment 10: Testing of Hydrogen Production Rate of Rod-like Magnesium-Platinum Metal Composite Structure obtained in Reference-embodiment 9

In order to explore the hydrogen production capacity of the rod-like magnesium-platinum metal composite structure, the rod-like magnesium-platinum metal composite structure prepared in Reference-embodiment 9 and a simple magnesium rod, both of which had the same size, were respectively placed in 50 mL of phosphate buffer; and further, the hydrogen yields of the rod-like magnesium-platinum metal composite structure and the simple magnesium rod were quantitatively determined at different times by gas chromatography.

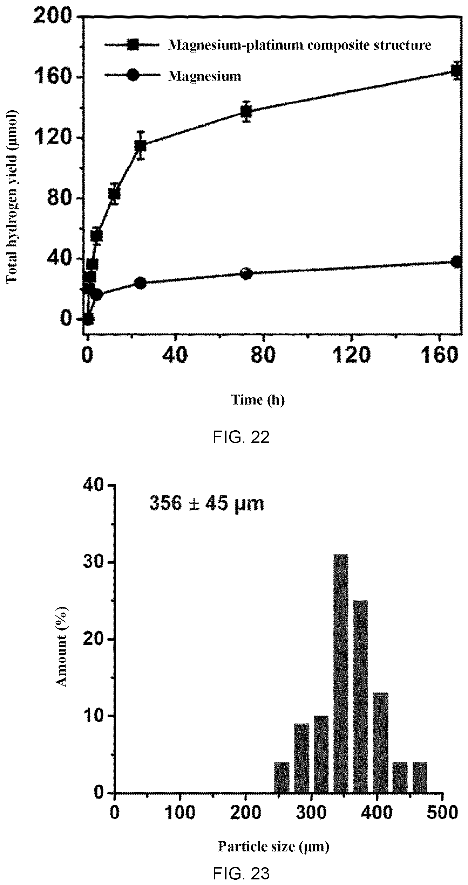

The curve graph of the determined total hydrogen yields was shown as in FIG. 22. As can be seen from the results in FIG. 22, after the magnesium rod was laced in the buffer, a small amount of hydrogen could be produced only at the very beginning, while the rod-like magnesium-platinum metal composite structure could produce a large amount of hydrogen continuously for a longer time, with much greater hydrogen production rate and total yield than those of the simple magnesium rod. These experimental results directly demonstrated that the magnesium-platinum metal composite structure of the present disclosure had excellent hydrogen production capacity and showed the potential for further use in tumor treatment.

### Reference-embodiment 11: Composite Embolic Agent

A composite embolic agent comprising the magnesium-platinum metal composite structure of Embodiment 9 and lipiodol.

The rod-like metal composite structure can be adjusted in the aspect ratio to achieve better fixation at the action site, thereby avoiding vascular blockage of other normal tissues caused by the metal composite structure entering the blood circulation when the composite embolic agent including the rod-like magnesium-platinum metal composite structure and lipiodol were used.

### Reference-embodiment 12: Aluminum-Gallium Metal Composite Structure

An aluminum-gallium metal composite structure comprising an aluminum core structure and gallium particles bound to the surface of the aluminum core structure. A method for preparing the aluminum-gallium metal composite structure is as follows.

At room temperature, 200 mg of aluminum powder (300 mesh, about 48 microns) was placed in a 4 mL centrifuge tube, to which 200 mg of liquid metal gallium was added dropwise and magnetically stirred for 10 min to prepare the aluminum-gallium metal composite structure by utilizing the strong fusion capacity of gallium to aluminum. After stirring, a produce was washed with absolute ethanol several times, and then sealed and preserved in absolute ethanol for future use.

### Reference-embodiment 13: Composite Embolic Agent

A composite embolic agent comprising the aluminum-gallium metal composite structure of Reference-embodiment 12 and lipiodol. The aluminum-gallium metal composite structure was mixed with the lipiodol to obtain the aluminum-gallium metal composite structure, which was allowed to be more uniformly dispersed in the lipiodol by an ultrasonic method. Metal aluminum reacted with water to produce aluminum hydroxide, which could be used as an immune adjuvant to regulate the immune microenvironment of the tumor site and enhance the effect of immunotherapy.

### Reference-embodiment 14: Composite Embolic Agent of Alloy Metal Particles

A composite embolic agent of alloy metal particles comprising platinum-gold alloy metal particles and lipiodol. The platinum-gold alloy metal particles were commercially purchased. The alloy metal particles could be further doped with other ions to possibly achieve a function of providing trace elements.

For example, the surfaces of the platinum metal particles were exposed to zinc, iron, manganese to obtain the composite embolic agent of alloy metal particles and lipiodol.

### Embodiment 15

A large-size metal microsphere (specifically, a magnesium microsphere), with the particle size distribution as shown in FIG. 23, and the particle size of about 350 ± 45 microns.

### Embodiment 16

Testing of the effect of the large-size microspheres described in Embodiment 15 on hydrogen production in buffers under different pH conditions. 20 mg of magnesium microspheres were placed in 4 mL of phosphate buffers with different pH values, and the cumulative hydrogen content arising from the reaction of magnesium microspheres in the buffers with different concentrations was quantitatively determined by gas chromatography.

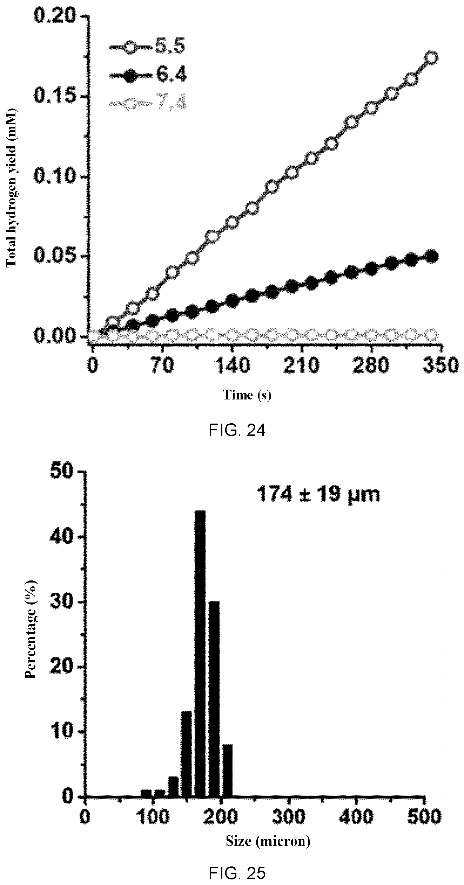

The measurement results of hydrogen produced by the magnesium microspheres with the particle size of about 350 ± 45 microns at different pH values were shown in FIG. 24. With pH=7.4, there was almost no hydrogen produced in the buffer; with pH=6.4, hydrogen was produced but at low yield and low rate compared with the condition of pH=7.4; and with pH=5.5, after reaction for 350 seconds, the cumulative hydrogen content arising from the magnesium microspheres reached about 3.5 times that in case of pH=6.4. This indicated that the magnesium microspheres could produce hydrogen in a slightly acidic environment, and as the pH value decreased, the hydrogen production rate increased. This suggested that the magnesium microspheres with large particle size could produce hydrogen in the slightly acidic physiological environment of tumors, and showed the potential for further use in hydrogen treatment of tumors.

### Embodiment 17

A composite embolic agent containing active metal microsphere with large particle size, comprising the magnesium microspheres with large particle size (about 350 ± 45 microns) described in Embodiment 15 and a lipiodol embolic agent (commercially available). The magnesium microspheres with large particle size and the lipiodol were mixed, and the mass ratio of the magnesium microspheres to the lipiodol was 10:100. A uniformly dispersed emulsion was obtained in an ultrasonic manner.

The magnesium-platinum composite structure could produce hydrogen in a physiological solution under a neutral pH condition, and the active metal microspheres could produce hydrogen in a physiological solution under a slightly acidic pH condition. The composite embolic agent formed by such microspheres and lipiodol could deliver hydrogen during tumor embolization to enhance the tumor embolization.

### Embodiment 18

Magnesium microspheres with the particle size of about 175 microns, prepared by an ultrasonic atomization powdering method.

The microscopic morphology of the obtained magnesium microspheres was observed through a microscope, their particle size distribution was statistically obtained. As shown in FIG. 25, the range of the particle size of the magnesium microspheres is about 174 ± 19 µm. It indicates that the median particle size of the magnesium microspheres is about 174 µm.

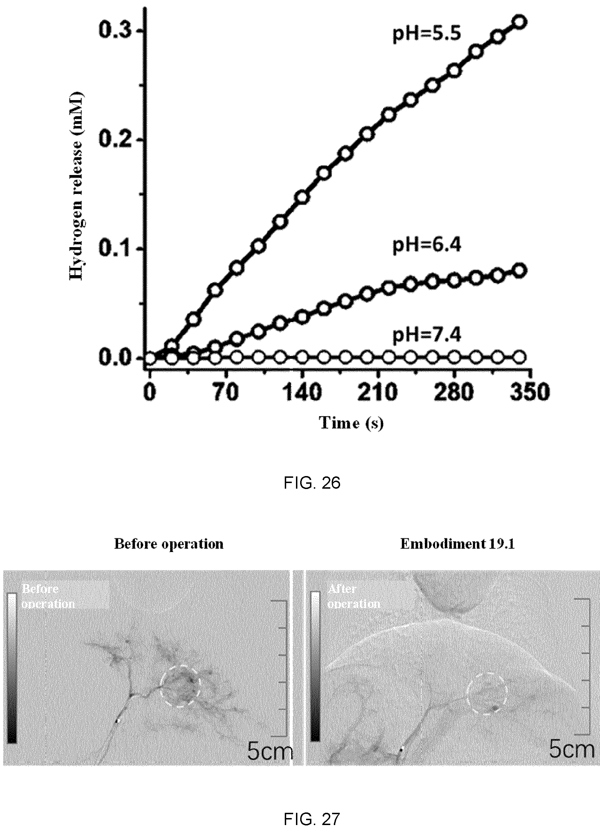

The hydrogen release rate of 5 mg magnesium microspheres from Embodiment 18 in citric acid-sodium citrate buffers with different pH values (pH 5.5, 6.4, and 7.4) was measured. The concentration of hydrogen released at different time points was measured, with the statistical results shown in FIG. 26, in which the vertical axis indicates the concentration of hydrogen in mM, i.e., millimole per liter. The higher the amount of hydrogen released per unit time, the stronger the reaction of the magnesium microspheres. The results showed that, compared with the buffer of neutral pH (pH=7.4), the buffers of pH=6.4 and pH=5.5 allowed for the release of higher amount of hydrogen per unit time, which indicated that the hydrogen was more easily produced from the magnesium microspheres in a weakly acidic environment, which is more conducive to improve the microenvironment of the tumor site (5.5-6.5), thereby enhancing the effect of embolization.

### Embodiment 19: Application of Magnesium Microspheres in Embodiment 18 to Rabbit Liver Cancer Model for Embolization

With the guidance of CT imaging, the VX2 liver tumor tissue was directly embedded in the left lobe of the liver, and a rabbit in situ liver cancer model was established after two weeks. The left image in FIG. 27 shows the tissues of the established rabbit liver cancer model, which proves the establishment of the left liver lobe cancer model of tumor-bearing rabbit, in which the size of the left liver lobe cancer lesions in the tumor-bearing rabbit is 1.0-1.5 cm.

The tumor-bearing rabbits were randomly divided into two groups and treated separately. Embodiment 19.1: According to the solution in FIG. 8, the magnesium microspheres of Embodiment 18 were implanted into the liver cancer lesion site in rabbits for embolization.

Comparative Example 19.2 (not according to the claimed invention): The control group was not subjected any treatment.

The blood vessels at the lesion site were observed by the method, and the results shown in the right image in FIG. 27 indicated that the blood supply to the rabbit liver disappeared (compared with the left image, the blood vessels in angiogram became less lighter, indicating insufficient blood supply), suggesting that the liver arterial embolization was achieved by delivering the 175 µm magnesium microspheres of Embodiment 18 by femoral artery interventional operation.

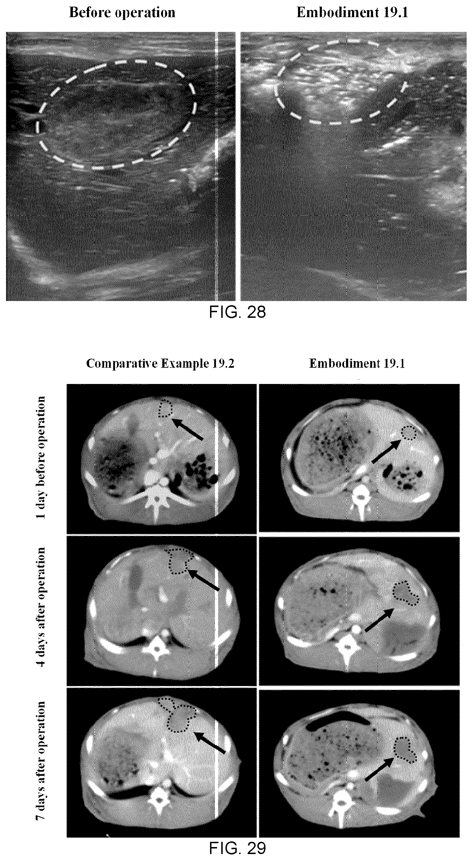

In addition, ultrasonic imaging was used to detect the gas echo at the liver lesion site, and the results are shown in FIG. 28. Comparing the conditions before (left image) and after (right image) operation, the site embolized by magnesium microspheres showed a stronger signal, i.e., stronger gas echo, demonstrating that the magnesium microspheres produced hydrogen at the embolized site. These demonstrated that the magnesium microspheres could produce hydrogen during embolization, thereby achieving the effect of hydrogen treatment.

On Day 4 and Day 7 after treatment, the efficacy was evaluated by using CT imaging enhanced with ioversol contrast agent, and the results are shown in FIG. 29, including the CT image data of Embodiment 19.1 and Comparative Example 19.2 at different times before and after treatment. The results showed that, in Comparative Example 19.2, the tumor volume on Day 4 increased by 2.5 times compared with the tumor volume on the day before treatment, and the tumor volume on Day 7 increased by 5.8 times compared with the tumor volume on the day before treatment. According to mRECIST, it was evaluated as a progressive disease (PD). The rabbit liver cancer lesion in Embodiment 19.1 changed slowly. The tumor volume on Day 4 increased by 2 times compared with the tumor volume on the day before the treatment, and the tumor volume on Day 7 increased by 1.8 times compared with the tumor volume on the day before the treatment. According to mRECIST, it was evaluated as an effective response.

Ki67 existed in the late phase G1 and the phases S, G2, and M in the cell cycle, but was not expressed in cells in the phase G0. Therefore, the signal intensity of Ki67 reflected the cell proliferation activity. The stronger the positive signal of Ki67, the higher the cell proliferation activity and the higher the degree of malignancy, leading to poor prognosis of patients. The tumor tissues were frozen, sliced, and stained with Ki67 for observation, and the Ki67 fluorescence intensity was statistically processed. It was found that the proliferative activity of the sample in Comparative Example 2 was much higher than that in Embodiment 19.1. It showed that the activity of rabbit liver cancer cells decreased after the embolization with the magnesium microsphere. That is, the embolization with the magnesium microspheres can effectively inhibit liver cancer, showing the potential for cancer treatment.

### Reference-embodiment 20: A manganese-platinum metal composite structure comprising a first metal manganese and a second metal platinum bound to the surface of the manganese.

The manganese-platinum metal galvanic cells composite structure was prepared by the replacement method as specified below.

At room temperature, 200 mg of manganese powder (1-5 µm) was dispersed in 10 mL of absolute ethanol solution; an absolute ethanol solution containing 0.5% sodium chloroplatinate (PtCl₆) with a mass fraction of 0.5% was added dropwise under magnetic stirring; and based on the principle of in-situ replacement reduction, the platinum nanoparticles were spontaneously reduced on the surface of micron-sized manganese particles, thereby preparing the manganese-platinum metal composite structure. After reacting for about 30 minutes, the manganese-platinum metal composite structure was collected by centrifugation, washed with absolute ethanol several times to remove unreacted ions on the surface, and sealed and preserved in absolute ethanol.

In the obtained manganese-platinum metal composite structure, based on the activity difference between the two metals of manganese and platinum, the platinum nanoparticles attached to the surface of the manganese metal acted as the positive electrode in the galvanic cells reaction, and the manganese was the positive electrode in the galvanic cells reaction, such that the manganese-platinum metal galvanic cells composite structure could spontaneously undergo the galvanic cells reaction in an aqueous solution to continuously produce hydrogen, thereby achieving the purpose of improving the tumor microenvironment and implementing hydrogen treatment.

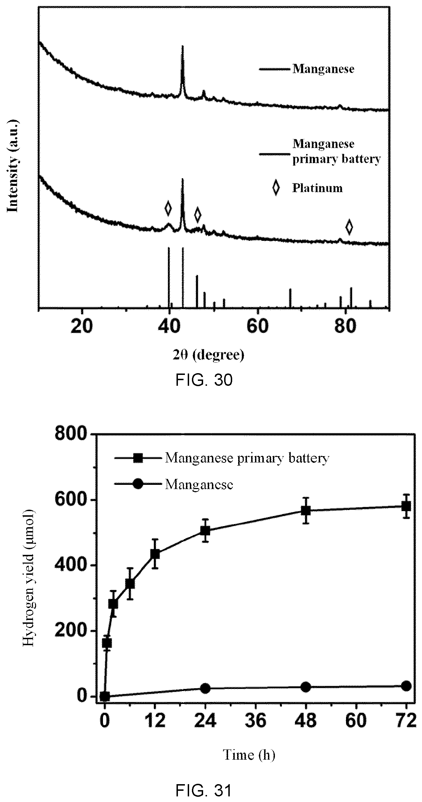

The elemental analysis was carried out on the prepared manganese-platinum metal composite structure by an X-ray diffractometer. FIG. 30 is the X-ray diffraction pattern of the manganese-platinum metal composite structure, in which the peaks of both metal manganese and platinum are identified, demonstrating that the metal platinum was attached to the surface of the metal manganese core through the redox reaction, forming the metal composite structure.

### Reference-embodiment 21: Experiment on In vitro Hydrogen Production Efficiency

The manganese-platinum metal composite structure prepared in Embodiment 20 was placed in phosphate buffer, and the content of hydrogen produced at different times was quantitatively detected by gas chromatography. FIG. 31 is the statistical graph of the content of hydrogen produced by the metal manganese and metal composite structure in the phosphate buffer at different time points. The results showed that the stability and continuity of hydrogen production by manganese in the phosphate buffer were low; while the manganese-platinum metal composite structure could produce hydrogen more stably and continuously, with higher total hydrogen yield. After 72 hours of reaction, the cumulative hydrogen content arising from the manganese-platinum metal composite structure reached 10 times that of the manganese. Therefore, the manganese-platinum metal composite structure with the inactive metal as the main core structure had better application potential in hydrogen treatment than inactive metal.

### Reference-embodiment 22: Verification of Therapeutic Effect of Manganese-Platinum Metal Composite Structure on Tumor Treatment in Mice

The manganese-platinum metal composite structure prepared in Reference-embodiment 20 was used to verify its ability to regulate the tumor microenvironment and produce hydrogen in a mouse subcutaneous colon cancer tumor model.

First, the mouse subcutaneous colon cancer tumor model was established by inoculating 3×10⁶ CT26 mouse colon cancer cells on the right back of each mouse; and when the volume of the subcutaneous colon cancer tumor of the mouse grew to about 150 mm³, the mice were randomly divided into 3 groups:
Embodiment 22.1: control group, without any treatment;
Embodiment 22.2: intratumoral injection of a micron-sized metal manganese particle suspension at a dose of 1.5 mg/mouse; and
Embodiment 22.3: intratumoral injection of a manganese-platinum metal composite structure suspension at a dose of 1.5 mg/mouse.

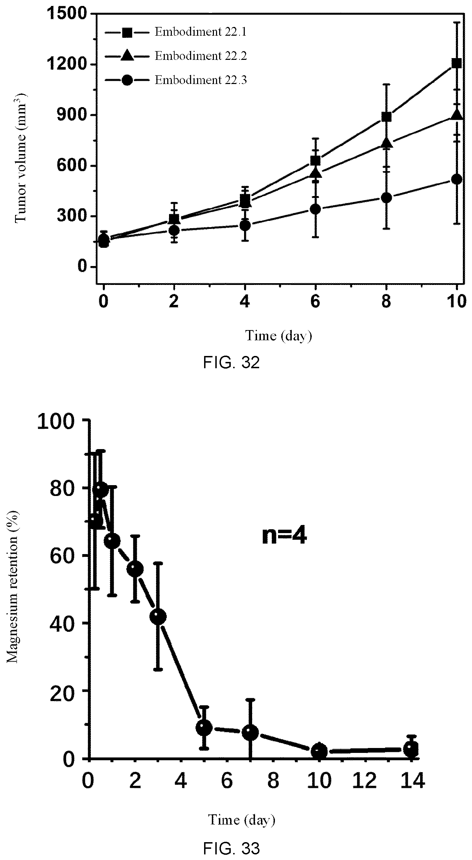

The manganese-based metal composite structure prepared by the method described in Embodiment 20 was injected into the tumor in each mouse, and after treatment by different means, the tumor volume was monitored every two days until the volume was greater than 1500 mm³, which was regarded as the monitoring endpoint. The results were shown in FIG. 32. In Embodiment 22.2, the growth of tumors in mice was only slightly inhibited, with little difference from the control group; while in Embodiment 22.3, the growth of tumors in mice was significantly inhibited. In conclusion, the manganese-platinum metal composite structure of the present disclosure is applicable to hydrogen treatment of tumors, with significantly improved efficacy compared with the hydrogen treatment by the micron-sized manganese particles.

### Embodiment 23: Retention of Lesion Site

Mice were subcutaneously inoculated with tumors, and when the tumor volume reached 60-80 mm³, the 40 mg/mL magnesium microsphere-lipiodol composite embolic agent prepared in Embodiment 1 was injected into each tumor at 50 mL per mouse; then the mice were sacrificed at different time points to remove the tumors, which were then digested with aqua regia; the Mg content in tissues was detected by ICP-OES; and the residual rate of magnesium in the tumor was calculated as the residual rate of magnesium = the mass of magnesium in the tumor/total dose used. FIG. 33 shows a statistical graph of residual magnesium at the mouse tumor site at different times after intratumoral injection of magnesium microsphere-lipiodol composite embolic agent.

The results in FIG. 33 showed that the magnesium microspheres could persist at the tumor site for a certain period of time (about 4 days), indicating that the composite embolic agent could residue at the lesion site for a long time to achieve the long-term effects of embolization and microenvironment regulation. As time goes on, the magnesium microspheres gradually decreased in content and could be effectively metabolized, indicating that the magnesium microspheres could be slowly degraded. That is, the magnesium microspheres could be catabolized in vivo, showing low safety risk to the human body.

### Embodiment 24: PH Regulation at Tumor Site

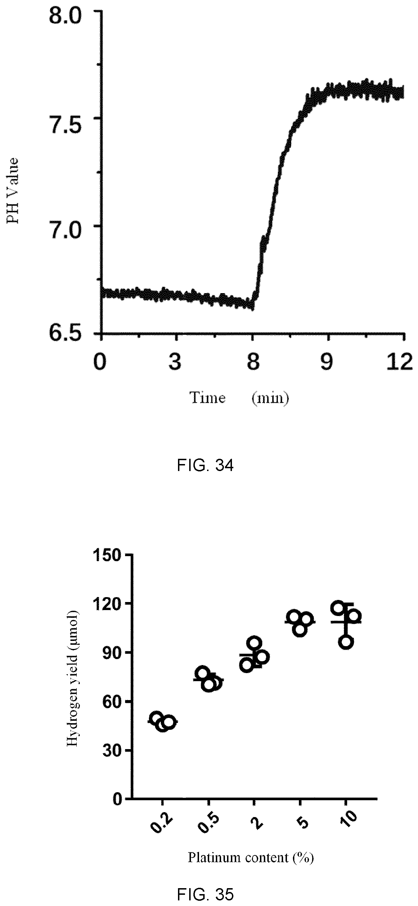

Mice were subcutaneously inoculated with tumors, and when the tumor volume reached 100-150 mm³, a pH electrode was inserted into each tumor to detect the intratumoral pH value. Subsequently, the mice were intratumorally injected with 50 mL of 40 mg/mL magnesium microsphere-lipiodol composite embolic agent prepared in Embodiment 1, and at the same time, the change of intratumoral pH was monitored by the pH electrode. FIG. 34 showed the change of pH in the mouse tumor over time in Embodiment 24. The results showed that before the injection of the magnesium microsphere-lipiodol composite embolic agent prepared in Embodiment 1, the tumor site had a weakly acidic pH environment, and the pH was maintained at about 6.7; and after the intratumoral injection of the magnesium microsphere-lipiodol composite embolic agent, the intratumoral pH increased to about 7.6 and sustained. It indicated that the magnesium microsphere composite embolic agent prepared in Embodiment 1 had the ability to regulate the intratumoral acidic microenvironment and improve the weakly acidic environment at the tumor site. The lower pH of the tumor site promoted tumor progression to affect the efficacy of tumor treatment, and formed an inhibitory immune microenvironment to the tumor immune escape. The magnesium microsphere-lipiodol composite embolic agent prepared in Embodiment 1 improved the acidic microenvironment at the tumor site and could better inhibit tumor growth; and the improved acidic microenvironment at the tumor site could reduce the drug resistance of tumor, thereby also helping combined chemotherapy, radiotherapy, immunotherapy and other therapies to improve the efficacy of tumor treatment.

### Reference-embodiment 25: Hydrogen Production Efficiency of Magnesium-Platinum Metal Composite Structures with Different Metal Ratios

The commercial magnesium rod was placed in the ethanol solution containing sodium chloroplatinate to conduct the replacement reaction for 1 min at room temperature to obtain the magnesium-platinum metal composite structure; and the concentration of the sodium chloroplatinate solution was changed, by which the magnesium-platinum metal composite structures with different ratios could be obtained by changing the feed ratio, since the replacement reaction occurred more fully and rapidly. Here, the magnesium rod had the short diameter of about 0.5 mm, and the long diameter of about 2 mm. The size of the obtained magnesium-platinum metal composite structure was similar to that of the magnesium rod, and only a small amount of metal platinum was attached to the surface of the magnesium rod.

4 mg of the magnesium-platinum metal composite structure was placed into 50 mL of phosphate buffer with pH = 6.5, and reacted in a sealed manner for 24 hours. The contents of hydrogen produced by the metal composite structures with different metal ratios were detected by gas chromatography. FIG. 35 showed the statistical graph of the contents of hydrogen produced by the magnesium-platinum metal composite structures with different ratios in Reference-embodiment 25.1 in a solution within 24 hours (the mass fractions of the metal platinum in the metal magnesium in Reference-embodiment 25 were 0.2%, 0.5%, 2%, 5%, and 10% respectively), with the vertical axis indicating the total yield of hydrogen produced in the system within about 24 h, and the abscissa axis indicating the mass fraction of the platinum in the magnesium rod in the magnesium-platinum metal composite structure.

From FIG. 35, it could be seen that, as the mass fraction of the platinum in the magnesium rod increased, the total yield of hydrogen produced within 24 hours gradually increased; when the mass fraction of the platinum reached 5%, the total yield of the hydrogen produced within 24 hours tended to be stable; and when the mass fraction of the platinum in the magnesium was greater than 5%, the total yield of the hydrogen produced within 24 hours no longer changed significantly. It suggested that the ratio of the two metals in the magnesium-platinum metal composite structure affected the hydrogen production efficiency of the magnesium-platinum metal composite structure. The mass fraction of the platinum in the magnesium might range from 0.2% to 10%, preferably from 0.2% to 5%. Overhigh platinum content was not conducive to greatly increase the hydrogen production efficiency, but would increase the in vivo content of metal elements, leading to increased metabolic burden and increased production cost. Therefore, an appropriate range of ratio could ensure the efficiency of hydrogen production and control the production cost, and also further improve the safety to human bodies.

### Reference-embodiment 26: Hydrogen Production Efficiency of Manganese-Platinum Metal Composite Structures with Different Metal Ratios

The commercial manganese powder (with the particle size of about 2.5 µm) was placed in the ethanol solution of sodium chloroplatinate, and undergone replacement reaction for 10 min at room temperature to obtain the manganese-platinum metal composite structure; the manganese-platinum metal composite structures with different metal ratios could be obtained by changing the concentration of the sodium chloroplatinate solution, thereby changing the feed ratio. Here, the manganese powder had the particle size of about 2.5 µm, and after the metal platinum was attached, the particle size distribution of the manganese powder was 2.5-5.5 µm.

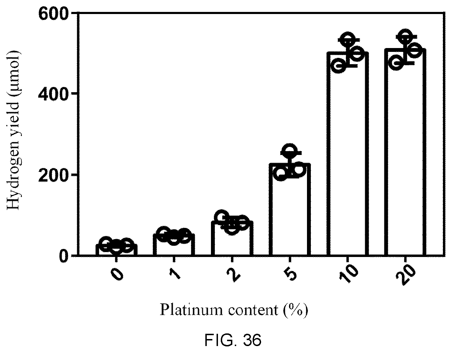

40 mg of the manganese-platinum metal composite structure was placed into 50 mL of phosphate buffer with pH = 6.5, and reacted in a sealed manner for 24 hours. The contents of hydrogen produced by the metal composite structures with different metal ratios were detected by gas chromatography. FIG. 36 showed the statistical graph of the contents of hydrogen produced by the manganese-platinum metal composite structures with different metal ratios in Reference-embodiment 25.2 in a solution within 24 hours (the mass fractions of the platinum in the manganese were 0, 1%, 2%, 5%, 10%, and 20% respectively).

The vertical axis indicated the total yield of hydrogen produced in the system within about 24 h, and the abscissa axis indicated the mass ratio of the platinum to the manganese powder in the manganese-platinum metal composite structure.

From FIG. 36, it could be seen that, as the mass ratio of the platinum to the micron-scale manganese particles increased,
the total yield of hydrogen produced within 24 hours gradually increased; when the mass fraction of the platinum reached 10%, the total yield of the hydrogen produced within 24 hours tended to be stable; and when the mass fraction of the platinum in the manganese was greater than 10%, the total yield of the hydrogen produced within 24 hours no longer changed significantly. It suggested that the ratio of the two metals in the manganese-platinum metal composite structure affected the hydrogen production efficiency of the manganese-platinum metal composite structure. The mass fraction of the platinum in the manganese might range from 1% to 20%, preferably from 1% to 10%. Overhigh platinum content was not conducive to greatly increase the hydrogen production efficiency, but would increase the in vivo content of metal elements, leading to increased metabolic burden and increased production cost. Therefore, an appropriate range of ratio could ensure the efficiency of hydrogen production and control the production cost, and also further improve the safety to human bodies.

### Reference-embodiment 27: Hydrogen Production Efficiency of Other Alloy Microspheres

In case of the customized magnesium-platinum alloy metal microspheres, with the increase of the particle size of the magnesium-platinum alloy metal microspheres and the decrease of platinum content, the hydrogen production efficiency gradually decreased. This was probably because less platinum was distributed on the surfaces of the alloy metal structure microspheres when the platinum content decreased. Compared with the metal composite structure, the customized magnesium-platinum alloy metal microspheres showed reduced efficiency of galvanic cells interaction on the surface and decreased hydrogen production rate. However, the alloy metal microspheres could still produce hydrogen, showing the potential for further use in tumor embolization.

We hope to produce more hydrogen at the lesion site to achieve better results, but the amount of hydrogen dissolved in the tissue and physiological environment is limited. Hence, the dose, administration frequency or other parameters can be changed in clinical practice to meet the requirements of treatment. Therefore, the metal microspheres, alloy metal microspheres, and metal composite structures of different sizes can be used as the components of composite embolic agent for hydrogen treatment to achieve tumor treatment.

Apparently, the above-mentioned embodiments are only for clear description, and are not intended to limit the embodiments.

Although the above disclosure has discussed, by way of various examples, some embodiments of the present disclosure that are presently considered to be useful, it should be understood that such details are for illustrative purposes only and that the appended claims are not limited to the disclosed embodiments.

Finally, it should be understood that the embodiments described in the present application are only intended to illustrate the principles of the embodiments of the present application. Other variations may also fall within the scope of the present application. Therefore, by way of example instead of limitation, the alternative configurations of the embodiments of the present application may be considered consistent with the teachings of the present application.

## Claims

1. A lipiodol-based composite embolic agent of active metal microspheres or nano-hydrides, **characterized in that** it comprises active metal microspheres or nano-hydride materials and lipiodol, wherein the active metal microspheres or the nano-hydride materials account for a mass percentage of 0.1-10% in the composite embolic agent, wherein
the active metal microspheres have a particle size of 0.1-20 microns, and the active metal is one or more of magnesium, aluminum, zinc, gallium, manganese and tin;
or
the nano-hydride materials have a particle size of 5-200 nanometers, and the nano-hydride is one or more of calcium hydride, magnesium hydride, lithium hydride, sodium hydride, potassium hydride, strontium hydride and cerium hydride.

2. A method for preparing the composite embolic agent as defined in claim 1, **characterized in that** it comprises steps of: mixing and uniformly dispersing lipiodol and active metal microspheres or nano-hydride materials to obtain the composite embolic agent.

3. The composite embolic agent according to claim 1 for use in liver cancer embolization.

4. A composite embolic agent, **characterized in that** it comprises an active metal microsphere, wherein the active metal microsphere has a metal activity stronger than that of hydrogen; the active metal microsphere has a particle size of 0.1-500 microns;
the active metal microsphere is prepared from an active metal, which has a metal activity greater than that of hydrogen and which is selected from at least one of magnesium, aluminum, zinc, gallium, iron, manganese, and tin, wherein the composite embolic agent further comprises a dispersant, wherein a mass ratio of the active metal microspheres to the dispersant is 0.1:100-50:100.

5. The composite embolic agent according to claim 4, **characterized in that** the dispersant is an organic phase.

6. The composite embolic agent according to claim 4, **characterized in that** the dispersant is an oil phase.

7. The composite embolic agent according to any one of claims 4-6, **characterized in that** the dispersant is selected from one or more of lipiodol, poppyseed oil, soybean oil, and olive oil.

8. The composite embolic agent according to claim 5, **characterized in that** the active metal microsphere is shaped as a sphere, a porous structure, or a polyhedral structure.

9. An anti-tumor agent, comprising an active metal microsphere, wherein the active metal microsphere has a metal activity stronger than that of hydrogen; the active metal microsphere has a particle size of 0.1-500 microns; the active metal microsphere is prepared from an active metal, which has a metal activity greater than that of hydrogen and which is selected from at least one of magnesium, zinc, gallium, manganese, and tin.

## Patentansprüche

1. Ein lipiodolbasiertes Komposit-Embolisationsmittel aus Aktivmetallmikrosphären oder Nanohydriden, **dadurch gekennzeichnet, dass** es Aktivmetallmikrosphären oder Nanohydrid-Materialien und Lipiodol umfasst, wobei die Aktivmetallmikrosphären oder die Nanohydrid-Materialien einen Massenprozentanteil von 0,1-10% in dem Komposit-Embolisationsmittel ausmachen, wobei
die Aktivmetallmikrosphären eine Partikelgröße von 0,1-20 Mikrometern aufweisen und das Aktivmetall eines oder mehrere von Magnesium, Aluminium, Zink, Gallium, Mangan und Zinn ist;
oder
die Nanohydrid-Materialien eine Partikelgröße von 5-200 Nanometern aufweisen und das Nanohydrid eines oder mehrere von Calciumhydrid, Magnesiumhydrid, Lithiumhydrid, Natriumhydrid, Kaliumhydrid, Strontiumhydrid und Ceriumhydrid ist.

2. Ein Verfahren zur Herstellung des Komposit-Embolisationsmittels wie in Anspruch 1 definiert, **dadurch gekennzeichnet, dass** es Schritte umfasst von: Mischen und gleichmäßigem Dispergieren von Lipiodol und Aktivmetallmikrosphären oder Nanohydrid-Materialien, um das Komposit-Embolisationsmittel zu erhalten.

3. Das Komposit-Embolisationsmittel nach Anspruch 1 zur Verwendung bei der Embolisation von Leberkrebs.

4. Ein Komposit-Embolisationsmittel, **dadurch gekennzeichnet, dass** es eine Aktivmetallmikrosphäre umfasst, wobei die Aktivmetallmikrosphäre eine Metallaktivität größer als die von Wasserstoff aufweist; die Aktivmetallmikrosphäre eine Partikelgröße von 0,1-500 Mikrometern aufweist; die Aktivmetallmikrosphäre aus einem Aktivmetall hergestellt ist, das eine Metallaktivität größer als die von Wasserstoff aufweist und das aus wenigstens einem von Magnesium, Aluminium, Zink, Gallium, Eisen, Mangan und Zinn ausgewählt ist, wobei das Komposit-Embolisationsmittel ferner ein Dispergiermittel umfasst, wobei ein Massenverhältnis der Aktivmetallmikrosphären zu dem Dispergiermittel 0,1:100-50:100 beträgt.

5. Das Komposit-Embolisationsmittel nach Anspruch 4, **dadurch gekennzeichnet, dass** das Dispergiermittel eine organische Phase ist.

6. Das Komposit-Embolisationsmittel nach Anspruch 4, **dadurch gekennzeichnet, dass** das Dispergiermittel eine Ölphase ist.

7. Das Komposit-Embolisationsmittel nach einem der Ansprüche 4-6, **dadurch gekennzeichnet, dass** das Dispergiermittel aus einem oder mehreren von Lipiodol, Mohnsamenöl, Sojabohnenöl und Olivenöl ausgewählt ist.

8. Das Komposit-Embolisationsmittel nach Anspruch 5, **dadurch gekennzeichnet, dass** die Aktivmetallmikrosphäre die Form einer Kugel, einer porösen Struktur oder einer polyedrischen Struktur aufweist.

9. Ein Antitumormittel, umfassend eine Aktivmetallmikrosphäre, wobei die Aktivmetallmikrosphäre eine Metallaktivität größer als die von Wasserstoff aufweist; die Aktivmetallmikrosphäre eine Partikelgröße von 0,1-500 Mikrometern aufweist; die Aktivmetallmikrosphäre aus einem Aktivmetall hergestellt ist, das eine Metallaktivität größer als die von Wasserstoff aufweist und das aus wenigstens einem von Magnesium, Zink, Gallium, Mangan und Zinn ausgewählt ist.

## Revendications

1. Un agent embolique composite à base de lipiodol à microsphères métalliques actives ou nano-hydrures, **caractérisé en ce qu'**il comprend des microsphères métalliques actives ou des matériaux de nano-hydrure et du lipiodol, dans lequel les microsphères métalliques actives ou les matériaux de nano-hydrure représentent un pourcentage massique de 0,1-10% dans l'agent embolique composite, dans lequel
les microsphères métalliques actives ont une taille de particules de 0,1-20 micromètres, et le métal actif est un ou plusieurs parmi le magnésium, l'aluminium, le zinc, le gallium, le manganèse et l'étain;
ou
les matériaux de nano-hydrure ont une taille de particules de 5-200 nanomètres, et le nano-hydrure est un ou plusieurs parmi l'hydrure de calcium, l'hydrure de magnésium, l'hydrure de lithium, l'hydrure de sodium, l'hydrure de potassium, l'hydrure de strontium et l'hydrure de cérium.

2. Un procédé de préparation de l'agent embolique composite tel que défini à la revendication 1, **caractérisé en ce qu'**il comprend les étapes consistant à: mélanger et disperser uniformément le lipiodol et des microsphères métalliques actives ou des matériaux de nano-hydrure afin d'obtenir l'agent embolique composite.

3. L'agent embolique composite selon la revendication 1 pour une utilisation dans l'embolisation du cancer du foie.

4. Un agent embolique composite, **caractérisé en ce qu'**il comprend une microsphère métallique active, dans lequel la microsphère métallique active a une activité métallique supérieure à celle de l'hydrogène; la microsphère métallique active a une taille de particules de 0,1-500 micromètres; la microsphère métallique active est préparée à partir d'un métal actif, lequel a une activité métallique supérieure à celle de l'hydrogène et lequel est sélectionné parmi au moins un de magnésium, aluminium, zinc, gallium, fer, manganèse et étain, dans lequel l'agent embolique composite comprend en outre un dispersant, dans lequel un rapport massique des microsphères métalliques actives au dispersant est de 0,1:100-50:100.

5. L'agent embolique composite selon la revendication 4, **caractérisé en ce que** le dispersant est une phase organique.

6. L'agent embolique composite selon la revendication 4, **caractérisé en ce que** le dispersant est une phase huileuse.

7. L'agent embolique composite selon l'une quelconque des revendications 4-6, **caractérisé en ce que** le dispersant est sélectionné parmi un ou plusieurs de lipiodol, huile de graines de pavot, huile de soja et huile d'olive.

8. L'agent embolique composite selon la revendication 5, **caractérisé en ce que** la microsphère métallique active est de forme sphérique, de structure poreuse ou de structure polyédrique.

9. Un agent antitumoral, comprenant une microsphère métallique active, dans lequel la microsphère métallique active a une activité métallique supérieure à celle de l'hydrogène; la microsphère métallique active a une taille de particules de 0,1-500 micromètres; la microsphère métallique active est préparée à partir d'un métal actif, lequel a une activité métallique supérieure à celle de l'hydrogène et lequel est sélectionné parmi au moins un de magnésium, zinc, gallium, manganèse et étain.
